# EUROPÄISCHE PATENTANMELDUNG

(11) **EP 4 485 308 A1**
(43) Veröffentlichungstag der Anmeldung: **01.01.2025**
(21) Anmeldenummer: 23182292.5
(22) Anmeldetag: 29.06.2023
(51) Int. Cl.: G06Q 10/087, G16H 40/40, A61C 13/00

(54) **DISKMANAGER FÜR DENTALE ROHLINGE**

(71) Anmelder: Ivoclar Vivadent AG, 9494 Schaan (LI)
(72) Erfinder: Cetin, Tarkan, 6800 Feldkirch (AT); Gassner, Sebastian, 9497 Triesenberg (LI); Gantioler, Andreas, 6700 Bludenz (AT); Bertagnolli, Martin, 39025 Naturns (IT)
(74) Vertreter: Baldus, Oliver

(57) **Zusammenfassung**

Die vorliegende Erfindung betrifft einen Diskmanager (100) zum Verwalten von dentalen Rohlingen (101-1, ..., 101-n), mit mehreren Aufnahmefächern (103-1, ..., 103-n) zum Aufnehmen jeweils eines dentalen Rohlings (101-1, ..., 101-n); und einer Anzeigevorrichtung (105-1, ..., 105-n) zum Anzeigen des Aufnahmefachs (103-1, ..., 103-n), in dem ein dentaler Rohling (101-1, ..., 101-n) aufgenommen ist.

## Beschreibung

Die vorliegende Erfindung betrifft einen Diskmanager zum Verwalten von dentalen Rohlingen, ein Modul für einen Diskmanager mit zumindest einem Aufnahmefach zum Aufnehmen eines dentalen Rohlings und ein Verfahren zum Verwalten von dentalen Rohlingen.

Das Auffinden von scheibenförmigen oder blockförmigen dentalen Rohlingen ist im zahntechnischen Arbeitsablauf oft mit einem Suchaufwand verbunden. Nachdem das richtige Material für eine dentale Restauration gewählt worden ist, kann zwischen verschiedenen dentalen Rohlingen vom selben Typ ausgewählt werden, aus denen zuvor bereits dentale Restaurationen herausgearbeitet worden sind. Je mehr dentale Rohlinge verfügbar sind, desto länger dauert die Suche nach dem korrekten Rohling.

Wenn nicht derjenige dentale Rohling mit der geringsten Menge Restmaterial gewählt wird oder neue dentale Rohlinge verwendet werden, bevor verfügbares Restmaterial verbraucht ist, wird der Lagerbestand unnötig erhöht. Zusätzlich zu dem Auswählen und Suchen der dentalen Rohlinge, entsteht ein Zeitaufwand für das Einsetzen und Herausnehmen der dentalen Rohlinge in die Diskhalter.

Beim Nesting oder Aktivieren neuer dentaler Rohlinge werden diese zunächst an einem Computer genestet, bevor diese in die Herstellungsmaschine geladen werden. Falls der Computer und die Herstellungsmaschine räumlich voneinander getrennt sind, entsteht ein weiterer Zeitverlust.

Durch Stapeln oder Lagern der Rohlinge auf Tischen, in Schubladen oder Regalen kann es durch häufiges Hantieren zu Brüchen an den Rändern der Rohlinge kommen. Nachdem ein dentaler Rohling vollständig abgenutzt ist, wird dieser entsorgt und ein Neuer nachbestellt. Der Restbestand des jeweiligen Materials im Lager hat zudem einen Einfluss auf den Bestellzeitpunkt und eine Bestellempfehlung.

Es ist die technische Aufgabe der vorliegenden Erfindung, den Handhabungsaufwand von dentalen Rohlingen zu verringern.

Diese Aufgabe wird durch Gegenstände nach den unabhängigen Ansprüchen gelöst. Technisch vorteilhafte Ausführungsformen sind Gegenstand der abhängigen Ansprüche, der Beschreibung und der Zeichnungen.

Gemäß einem ersten Aspekt wird die technische Aufgabe durch einen Diskmanager zum Verwalten von dentalen Rohlingen gelöst, mit mehreren Aufnahmefächern zum Aufnehmen jeweils eines dentalen Rohlings; und einer Anzeigevorrichtung zum Anzeigen des Aufnahmefachs, in dem ein dentaler Rohling mit aufgenommen ist. Der Rohling kann blockförmig oder scheibenförmig sein. Durch den Diskmanager kann der Suchaufwand für dentale Rohlinge verringert werden, nachdem von der CAM-Software oder der Herstellungsmaschine ein Auftrag geschickt wurde, da lediglich nach demjenigen Rohling gegriffen wird, der von der Anzeigevorrichtung angezeigt wird, beispielsweise dort wo eine Leuchtdiode leuchtet (Pick-by-Light). Ein Berührungskontakt mit dem Rohling erfolgt nur, wenn der Rohling tatsächlich benötigt wird. Auf ein loses Stapeln der Rohlinge kann verzichtet werden.

In einer technisch vorteilhaften Ausführungsform des Diskmanagers umfasst der Diskmanager eine Speichervorrichtung zum Speichern der Eigenschaften des dentalen Rohlings in dem jeweiligen Aufnahmefach. Die Speichervorrichtung ist beispielsweise ein digitaler Datenspeicher zum digitalen Speichern von Informationen über die Eigenschaften des Rohlings, wie ein RAM-Speicher oder eine Festplatte. Die Daten können auch online oder in einem Netzwerk gespeichert werden. Der Diskmanager kann auch eine Lese- und Schreibfunktion über RFID-Leser umfassen. Die ausgelesenen Daten können dann in einem Netzwerk weiterverarbeitet werden. Die Eigenschaften des dentalen Rohlings können Nestingdaten umfassen, in denen die Größe und Form der herausgefrästen Stellen angegeben ist, die zuvor aus dem dentalen Rohling herausgefräst worden sind. Durch die Nestingdaten kann bestimmt werden, ob noch weitere dentale Restaurationen aus dem jeweiligen Rohling herausgefräst werden können.

Dadurch wird beispielsweise der technische Vorteil erreicht, dass eine elektronische Lagerhaltung von dentalen Rohlingen durchgeführt werden kann.

In einer weiteren technisch vorteilhaften Ausführungsform des Diskmanagers umfassen die Eigenschaften ein verfügbares Restmaterial, eine Farbe, eine Identifikationsnummer, eine Größe und/oder ein Herstellungsmaterial des aufgenommenen Rohlings. Diese Informationen können in einer in einer CAM-Software gespeichert, verwaltet und ausgewertet werden. Dadurch wird beispielsweise der technische Vorteil erreicht, dass die Rohlinge anhand von besonders geeigneten Eigenschaften verwaltet werden können.

In einer weiteren technisch vorteilhaften Ausführungsform des Diskmanagers ist der Diskmanager aus mehreren Modulen mit zumindest einem Aufnahmefach zusammensetzbar. Dadurch wird beispielsweise der technische Vorteil erreicht, dass der Diskmanager an räumliche Gegebenheiten angepasst werden kann.

In einer weiteren technisch vorteilhaften Ausführungsform des Diskmanagers umfasst der Diskmanager eine Eingabeschnittstelle zum Eingeben von Eigenschaften eines dentalen Rohlings. Die Eingabeschnittstelle dient zum Einlesen des Rohlings. Der Diskmanager kann auch eine Eingabeschnittstelle zur Kommunikation mit einer CAM-Software oder einer Herstellungsmaschine umfassen. Dadurch wird beispielsweise der technische Vorteil erreicht, dass Daten über Rohlinge mit gesuchten Eigenschaften an den Diskmanager übermittelt werden können.

In einer weiteren technisch vorteilhaften Ausführungsform des Diskmanagers umfassen die Aufnahmefächer eine Neigung oder eine Schaumstoffeinlage zur stabilen Positionierung des dentalen Rohlings. Dadurch wird beispielsweise der technische Vorteil erreicht, dass ein unbeabsichtigtes Herausrollen des Rohlings verhindert werden kann.

In einer weiteren technisch vorteilhaften Ausführungsform des Diskmanagers umfasst der Diskmanager eine Scanvorrichtung zum Scannen eines dentalen Rohlings. Dadurch wird beispielsweise der technische Vorteil erreicht, dass Daten über Eigenschaften des Rohlings auf einfache Weise ermittelt werden können. Die Daten können über ein Netzwerk verglichen oder ausgetauscht werden.

In einer weiteren technisch vorteilhaften Ausführungsform des Diskmanagers umfasst die Scanvorrichtung eine optische Kamera, einen RFID-Leser, einen Bar-Code-Leser und/oder einen QR-Code-Leser. Dadurch wird beispielsweise der technische Vorteil erreicht, dass der Rohling auf einfache und schnelle Weise gescannt werden kann.

In einer weiteren technisch vorteilhaften Ausführungsform des Diskmanagers ist jedem Aufnahmefach eine Antenne oder ein Sensor zum Erfassen des aufgenommenen dentalen Rohlings zugeordnet. Die Antenne oder der Sensor erfassen, ob in dem Aufnahmefach ein dentaler Rohling aufgenommen ist. Der Sensor kann ein mechanischer Taster, ein Drucksensor oder ein Ultraschallsensor sein. Dadurch wird beispielsweise der technische Vorteil erreicht, dass eine aktuelle tatsächliche Belegung der Aufnahmefächer bekannt ist.

In einer weiteren technisch vorteilhaften Ausführungsform des Diskmanagers ist jedem Aufnahmefach eine Anzeigevorrichtung zugeordnet. Dadurch wird beispielsweise der technische Vorteil erreicht, dass das dem Rohling zugeordnete Aufnahmefach einfach und schnell erkannt werden kann.

In einer weiteren technisch vorteilhaften Ausführungsform des Diskmanagers umfasst die Anzeigevorrichtung eine Leuchtdiode, eine Flüssigkristallanzeige oder ein elektronisches Papier. Die Leuchtdiode kann farblich durchstimmbar sein. Dadurch wird beispielsweise der technische Vorteil erreicht, dass das dem Rohling zugeordnete Aufnahmefach eindeutig identifiziert werden kann.

Gemäß einem zweiten Aspekt wird die technische Aufgabe durch ein Modul für einen Diskmanager mit zumindest einem Aufnahmefach zum Aufnehmen eines dentalen Rohlings gelöst. Durch das Modul werden die gleichen technischen Vorteile wie durch den Diskmanager nach dem ersten Aspekt erreicht.

In einer technisch vorteilhaften Ausführungsform des Moduls umfasst das Modul eine Anzeigevorrichtung zum Anzeigen des Aufnahmefachs. Die Anzeigevorrichtung kann auch an dem Rohling gebildet sein. Dadurch wird beispielsweise der technische Vorteil erreicht, dass an dem Modul das einem Rohling zugeordnete Aufnahmefach angezeigt werden kann.

In einer weiteren technisch vorteilhaften Ausführungsform des Moduls ist das Modul horizontal oder vertikal koppelbar. Dadurch wird beispielsweise der technische Vorteil erreicht, dass der Diskmanager räumlich flexibel aufgebaut werden kann. Das ist für einen Diskmanager im Allgemeinen auch denkbar. Im Allgemeinen können die Module auch räumlich getrennt angeordnet sein. Nicht nur horizontal und vertikal, sondern auch räumlich getrennt.

Gemäß einem dritten Aspekt wird die technische Aufgabe durch ein Verfahren zum Verwalten von dentalen Rohlingen gelöst, mit den Schritten eines Aufnehmens eines dentalen Rohlings in einem von mehreren Aufnahmefächern; und eines Anzeigens des Aufnahmefachs, in dem ein dentaler Rohling mit vorgegebenen Eigenschaften aufgenommen ist. Durch das Verfahren werden die gleichen technischen Vorteile wie durch den Diskmanager nach dem ersten Aspekt erreicht.

In einer technisch vorteilhaften Ausführungsform des Verfahrens wird erfasst, ob ein dentaler Rohling in einem der Aufnahmefächer eingesetzt ist. Dadurch wird beispielsweise der technische Vorteil erreicht, dass eine Belegung der Aufnahmefächer ermittelt werden kann.

In einer weiteren technisch vorteilhaften Ausführungsform des Verfahrens wird die Restmenge eines dentalen Rohlings erfasst. Dadurch wird beispielsweise der technische Vorteil erreicht, dass eine Lagerbestand verwaltet werden kann.

In einer weiteren technisch vorteilhaften Ausführungsform des Verfahrens wird ein dentaler Rohling automatisch nachbestellt, wenn die Restmenge einen vorgegebenen Wert unterschreitet. Dadurch wird beispielsweise der technische Vorteil erreicht, dass eine ausreichende Zahl von jedes dentalen Rohlings vorhanden ist.

In einer weiteren technisch vorteilhaften Ausführungsform des Verfahrens wird ein Lagerbestand dentaler Rohlinge verwaltet. Dadurch wird beispielsweise der technische Vorteil erreicht, dass die dentalen Rohlinge derart verwaltet werden können, dass stets ausreichende Mengen vorhanden sind.

Ausführungsbeispiele der Erfindung sind in den Zeichnungen dargestellt und werden im Folgenden näher beschrieben.

Es zeigen:
- Fig. 1: eine schematische Ansicht eines Diskmanagers;
- Fig. 2: eine schematische Ansicht eines modular aufgebauten Diskmanagers; und
- Fig. 3: ein Blockdiagramm zum Verwalten von dentalen Rohlingen.

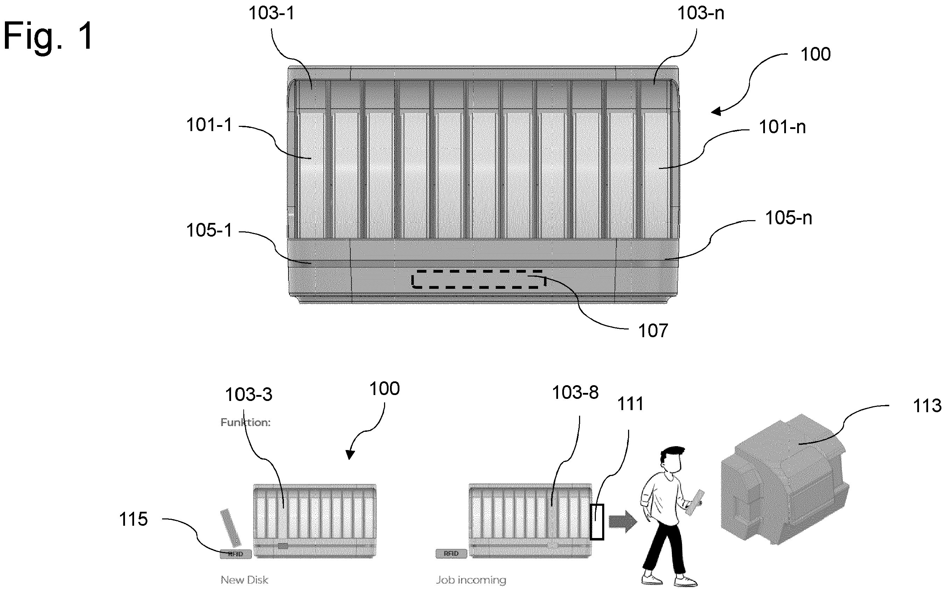

Fig. 1 zeigt eine schematische Ansicht eines Diskmanagers 100. Der Diskmanager 100 bildet ein strukturiertes Ablagesystem und umfasst mehrere Aufnahmefächer 103-1, ..., 103-n zum Aufnehmen jeweils eines dentalen Rohlings 101-1, ..., 101-n. Weiter umfasst der Diskmanager 100 eine Anzeigevorrichtung 105-1, ..., 105-n zum Anzeigen desjenigen Aufnahmefachs 103-1, ..., 103-n, in dem ein bestimmter dentaler Rohling 101-1, ..., 101-n aufgenommen ist.

Die Rohlinge 101-1, ..., 101-n können in den Aufnahmefächern 103-1, ..., 103-n einzeln gelagert werden. Die Form der Aufnahmefächer 103-1, ..., 103-n ist den Rohlingen 101-1, ..., 101-n angepasst. Beispielsweise schmiegt sich die hintere Wand an den Radius des Rohlings an. Mit einer Neigung rollen die Rohlinge 101-1, ..., 101-n sanft in die Aufnahmefächer 103-1, ..., 103-n und rollen nicht aus dem Diskmanager 100 heraus. Dies kann durch eine zusätzliche Kante oder Lippe oberhalb der Anzeigevorrichtung 105-1, ..., 105-n zusätzlich verhindert werden. Die Aufnahmefächer 103-1, ..., 103-n können mit Schaumstoff ausgestattet sein. Dadurch können Brüche der Rohlinge 101-1, ..., 101-n verhindert werden. Jedes Aufnahmefach 103-1, ..., 103-n kann eine eigene Antenne umfassen. Ein Antennenmultiplexer erkennt dadurch, welches Aufnahmefach 103-1, ..., 103-n belegt ist.

Der Diskmanager 100 umfasst eine digitale Speichervorrichtung 107 zum Speichern von Daten über die Eigenschaften des dentalen Rohlings 101-1, ..., 101-n, der in dem jeweiligen Aufnahmefach 103-1, ..., 103-n aufgenommen ist. Das Speichern von Daten über die Eigenschaften des dentalen Rohlings 101 1, ..., 101-n ist auch im Netzwerk möglich. Die Eigenschaften betreffen beispielsweise ein verfügbares Restmaterial, eine Farbe, eine Identifikationsnummer, eine Größe und/oder ein Herstellungsmaterial des aufgenommenen Rohlings. Zum Auswerten der gespeicherten Daten umfasst der Diskmanager 100 zudem einen Prozessor (CPU).

ROM, RAM und HD sind digitale Speichervorrichtungen 107 zum Speichern von Daten und computerausführbaren Anweisungen, die von dem Prozessor ausgeführt werden können oder kompiliert oder so interpretiert werden können, dass sie von dem Prozessor ausgeführt werden können. Geeignete computerausführbare Anweisungen können sich auf einem computerlesbaren Medium (z. B. ROM, RAM und/oder HD), Hardwareschaltkreisen oder dergleichen oder einer beliebigen Kombination davon befinden.

Die Speichervorrichtung 107 kann jede Art von Datenspeichermedium umfassen, das von einem Prozessor gelesen werden kann. Beispiele für computerlesbare Speichermedien können flüchtige und nichtflüchtige Computerspeicher und Speichervorrichtungen umfassen, sind aber nicht darauf beschränkt, wie z. B. Direktzugriffsspeicher, schreibgeschützte Speicher, Festplatten, Speichergeräte-Arrays mit direktem Zugriff, Flash-Speicherlaufwerke, optische Datenspeichervorrichtungen, schreibgeschützte Compact-Disc-Speicher und andere geeignete Computerspeicher und Datenspeichervorrichtungen. Die Speichervorrichtung 107 kann auch durch Funketiketten (RFID-Tags) auf den jeweiligen Rohlingen gebildet sein.

Der Diskmanager 100 kann einen oder mehrere Prozessoren umfassen, die mit einer oder mehreren Benutzereingabe- /Ausgabegeräten (E/A) und Speichervorrichtungen gekoppelt sind. Beispiele für E/A-Geräte können unter anderem Tastaturen, Displays, Monitore, Touchscreens, Drucker, elektronische Zeigegeräte wie Mäuse, Trackballs, Stifte, Touchpads oder dergleichen umfassen.

Der Diskmanager 100 kann mit einem Display, einem Informationsgerät und verschiedenen Peripheriegeräten wie Druckern, Plottern, Lautsprechern gekoppelt werden. Der Diskmanager 100 kann auch mit externen Computern oder anderen Geräten über eine Netzwerkschnittstelle, einen drahtlosen Transceiver oder eine andere Einrichtung gekoppelt sein, die mit einem Netzwerk gekoppelt ist, wie z. B. einem lokalen Netzwerk (LAN), Wide Area Network (WAN) oder dem Internet.

Über eine Eingabeschnittstelle 111 können Daten über die Eigenschaften eines gesuchten Rohlings 101 1, ..., 101-n zugeführt werden, wie beispielsweise über eine CAM-Software. Der Diskmanager 100 vergleicht diese Daten mit den gespeicherten Daten der aufgenommenen Rohlinge 101 1, ..., 101-n und zeigt mittels der Anzeigevorrichtung 105-1, ..., 105-n das Aufnahmefach 103-1, ..., 103-n an, in dem der Rohling 101 1, ..., 101-n mit den gewünschten Eigenschaften aufgenommen ist. Dadurch kann eine chaotische Ablage der Rohlinge erfolgen, da der Algorithmus in jedem Fall einen vorhandenen Rohling 101-1, ..., 101-n findet.

Eine Einsortierung der Rohlinge 101-1, ..., 101-n kann auch mit Struktur auf Basis von Sortieralgorithmen oder Machine-Learning-Algorithmen in einer digitalen Bibliothek durchgeführt werden. Der Diskmanager 100 kann auch einen Vorschlag für einen optimalen oder verschnitt-optimierten Rohling 101-1, ..., 101-n durchführen. Ein Filtern und Sortieren der Rohlinge 101-1, ..., 101-n kann in der CAM-Software erfolgen.

Zunächst kommt beispielsweise ein neuer Fräsauftrag von einer Herstellungsmaschine. Ist der dentale Rohling 101-8 mit dem gewünschten Material im Diskmanager 100 gelagert, so leuchtet das jeweilige Aufnahmefach 103-8 orange auf. Wenn der Rohling 101-8 aus dem Diskmanager 100 herausgenommen, in die Herstellungsmaschine 113 gelegt und von dieser erkannt wird, dann erlischt das jeweilige Aufnahmefach 103-8 am Diskmanager 100.

Wird in der Zwischenzeit ein neuer Rohling 101-3 eingesetzt, kann dieser am Diskmanager 100 über einen RFID-Leser als Scanvorrichtung 115 in einer CAM-Bibliothek, einer zentralen Datenbank oder auf einem Cloud-Server gespeichert werden und in ein grün leuchtendes Aufnahmefach 103-3 gelagert werden.

Ist der Auftrag abgeschlossen, so wird der Rohling 101-8 wieder aus der Herstellungsmaschine 113 herausgenommen, ebenfalls am RFID-Leser am Diskmanager 100 gescannt und dann wieder im grün leuchtenden Aufnahmefach 103-8 gelagert. Der Rohling 101-8 kann auch in einem beliebigen freien Aufnahmefach 103 oder in einem Aufnahmefach 103 gelagert werden, das über einen Sortier-Algorithmus gewählt wird. Dies kann auch willkürlich geschehen, da je nach Fräsauftrag die Rohlinge zu unterschiedlichen Zeiten aus einer Herstellungsmaschine herausgeführt werden.

Der Rohling 101-1, ..., 101-n kann auch während dem Einlegen des Rohlings 101-1, ..., 101-n in das Aufnahmefach 103-1, ..., 103-n gescannt werden. Hierzu kann ein Aufnahmefach 103-1, ..., 103-n mit einem integrierten Scanner (Scangate) verwendet werden, durch den der Rohling 101-1, ..., 101-n auf dem Weg in das Aufnahmefach 103-1, ..., 103-n gescannt wird. Eine Scanvorrichtung 115, die mit dem Diskmanager 100 gekoppelt ist, kann beweglich auf einer Schiene angeordnet sein. Die Scanvorrichtung 115 kann auch durch einen Barcode- oder QR-Code-Leser gebildet sein. Der Barcode oder QR-Code kann umlaufend um den Rohling 101-1, ..., 101-n herum angeordnet sein. Beim Scannen des Rohlings 101-1, ..., 101-n kann eine automatische Neubestellung durchgeführt werden. Ein Lieferschein kann in die CAM-Datenbank übernommen werden. Zudem kann eine digitale Lagerhaltung (digital Stockmanagement) oder eine Analyse und Auswertung von Nestingdaten durchgeführt werden.

Die Nestingdaten geben an, an welcher Stelle des dentalen Rohlings 101-1, ..., 101-n bereits dentale Restaurationen herausgefräst worden sind und wieviel Material hierbei verwendet worden ist.

Auf Basis der Nestingdaten kann bestimmt werden, an welcher Stelle und in welcher Größe weitere dentale Restaurationen aus dem Rohling 101-1, ..., 101-n herausgefräst werden können. Diese Nestingdaten können durch einen Informationsaustausch an einen Lagerschrank übermittelt werden.

Der Diskmanager 100 kann auch einen Drucker für Beschriftungsetiketten umfassen. Das Anlegen einer digitalen ID ist unabhängig von der Beschriftung. Die Beschriftung kann auf dem Diskhalter, dem Rohling oder Beschriftungsflaggen angeordnet sein. Hierzu können Farbcodes, Barcodes, oder QR-Codes verwendet werden.

Eine Eingabeschnittstelle 111 dient zum Eingeben von Eigenschaften eines dentalen Rohlings 101-1, ..., 101-n. Die Eingabeschnittstelle kann beispielsweise durch eine Datenverbindung mit einer CAM-Software gebildet sein oder durch eine Benutzerschnittstelle, über die ein Benutzer diese Daten manuell eingeben kann, wie beispielsweise einen berührungsempfindlichen Bildschirm. Die Eingabeschnittstelle 111 kann auch durch einen optischen Scanner gebildet sein, wie beispielsweise einen Bar-Code-Scanner oder einen QR-Code Scanner.

Der Rohling 101-1, ..., 101-n kann auf einem Wagen oder Halter gescannt und eingelagert werden (2 Achsenhandling). Zu diesem Zweck können RFID-Tags um den Rohling 101-1, ..., 101-n verteilt sein, so dass dieser von allen Seiten zu lesen ist. Die elektronische Eingabeschnittstelle 111 kann auch so ausgebildet sein, dass durch diese diejenigen Daten abgerufen werden, die auf dem RFID-Tag des Rohlings 101-1, ..., 101-n gespeichert sind.

Durch eine Kameraüberwachung des Schnittbildes des Rohlings 101 1, ..., 101-n von oben kann ein Abgleich mit der CAM-Datenbank und eine Erstellung einer Schnittbildbibliothek durchgeführt werden. Ein Einlesen des Rohlings 101 1, ..., 101-n kann direkt an der Herstellungsmaschine 113 oder am Diskmanager 100 durchgeführt werden.

Der Diskmanager 100 erzeugt einen Barcode, der mit der Herstellungsmaschine 113 synchronisiert wird. Die Barcode-Lese- und Druckstation kann direkt mit dem Diskmanager 100 gekoppelt werden. Analog zum Diskmanager 100 kann auch ein Blockmanager konzipiert werden, der in Matrixbauweise die Blöcke aufnimmt.

Durch den Diskmanager 100 wird eine Zeitersparnis im Herstellungsarbeitsablauf und eine Optimierung des Lagerbestands eines Dentallabors erreicht. Zudem wird eine Platzersparnis und ein übersichtlicheres Diskportfolio realisiert. Zudem ist eine Verlust- und Diebstahlüberwachung der Rohlinge 101-1, ..., 101-n durch den Diskmanager 100 möglich. Hierzu kann eine Identifizierung des Benutzers durch den Diskmanager 100 durchgeführt werden, wie beispielsweise durch eine Verriegelung oder personenbezogenes Anmelden.

Der verschnitt-optimierteste Rohling 101-1, ..., 101-n kann am Diskmanager 100 priorisiert werden, um den Lagerbestand optimal zu nutzen. Der Diskmanager 100 ermöglicht eine kompaktere Lagerung der Rohlinge 101-1, ..., 101-n gegenüber einer Schublade.

Der Diskmanager 100 weist einen portablen Formfaktor mit einer Stromversorgung über ein Netzteil, die Herstellungsmaschine 113, einen Computer (USB) oder eine Batterie auf. Der Diskmanager 100 kann auch als externer Diskwechsler mit integrierter Auftragskoordination und Stockmanagement gesehen werden. Der Diskmanager 100 kann als Stand-Alone-Diskmanager als Plugin für eine CAM-Software oder für eine Herstellungsmaschine konzipiert sein.

Mit der Anbindung des Diskmanagers 100 an eine Cloud kann verhindert oder ermöglicht werden, dass mehrere Herstellungsmaschinen 113 auf den gleichen Rohling 101 1, ..., 101-n zugreifen. Hierdurch können Zugriffskonflikte verhindert werden. Zudem kann der Diskmanager 100 neben der Herstellungsmaschine 113 stehen und den Transportweg vom Nesting am Computer zur Herstellungsmaschine 113 verkürzen. Neue Rohlinge 101 1, ..., 101-n, die sich noch in der Verpackung befinden, können direkt am Diskmanager 100 in die CAM-Software eingescannt werden. Durch intelligente Algorithmen werden die Daten erfasst und ausgewertet, sodass meistverwendete Materialien ermittelt werden und das optimale Aufnahmefach 103-1, ..., 103-n für den jeweiligen Rohling 101-1, ..., 101-n bestimmt wird.

Der Rohling 101-1, ..., 101-n kann auch in ein beliebiges Aufnahmefach 103-1, ..., 103-n eingelegt werden und der Diskmanager 100 erkennt durch einen Differenzabgleich, in welchem Aufnahmefach 103-1, ..., 103-n der Rohling 101-1, ..., 101-n eingelegt worden ist, beispielsweise mittels einer Antenne in jedem Fach.

Die Kommunikation über Eigenschaften der Rohlinge 101-1, ..., 101-n kann drahtlos oder drahtgebunden zwischen dem Diskmanager 100 und einer dentalen Herstellungsmaschine 113 erfolgen. Die Rohlinge 101-1, ..., 101-n werden nicht nur anhand des Materials vom Diskmanager 100 initialisiert, sondern auch mit dem aktuellen Fräsprofil, beispielsweise wieviel Raum für dentale Restaurationen noch auf dem Rohling 101-1, ..., 101-n verfügbar ist.

Dazu kommunizieren und synchronisieren sich die CAM-Software und der Diskmanager 100 miteinander, d.h. die Information über benutzte Rohlinge 101-1, ..., 101-n wird gespeichert und weitergeführt, bis diese entsorgt werden. Es kann ein Abgleich zwischen dem realen Rohling 101-1, ..., 101-n und einem digitalen Zwilling in der Datenbank (CAM-Bibliothek) vorgenommen werden.

Für das Verwalten und optimale Sortieren von Rohlingen kann der Diskmanager 100 mit einem Lager verbunden sein und mit der Herstellungsmaschine 113 und der CAM-Software interagieren. Der Benutzer erhält den optimalen Rohling 101-1, ..., 101-n für den jeweiligen Auftrag an der Herstellungsmaschine 113.

Abgekoppelt von der Herstellungsmaschine 113 kann die Kommunikation auch nur zwischen einer CAM-Software und dem Diskmanager 100 erfolgen. Dies ermöglicht es, den Diskmanager 100 an unterschiedliche Herstellungsmaschinen 113 anzuknüpfen und Fremdrohlinge zu lagern (offenes System).

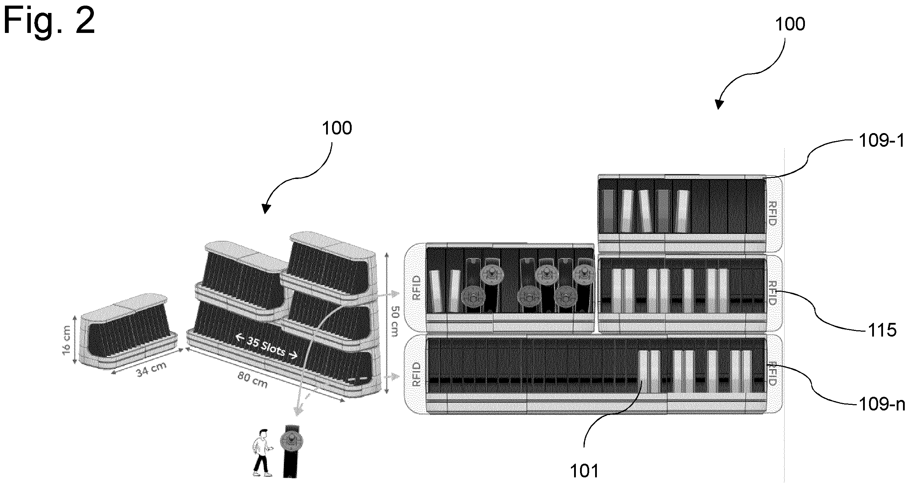

Fig. 2 zeigt eine schematische Ansicht eines modular aufgebauten Diskmanagers 100. Der Diskmanager 100 umfasst mehrere Module 109 mit Aufnahmefächern 103-1, ..., 103-n, die horizontal und/oder vertikal zusammensetzbar sind. Dieses Design ist platzsparender als Schubladen und in der Länge und Höhe skalierbar (C-Shape-Design). Auch das Stapeln von mehreren Diskmanagern 100 verschiedener Größen ist möglich. Eine Anzahl von Ein- und Ausspannvorgängen verringert sich bei einem Diskmanager 100 mit breiteren Aufnahmefächern 103-1, ..., 103-n.

Der Diskmanager 100 kann auf einem Unter- oder Überschrank an der Herstellungsmaschine in einer komfortablen Bedienhöhe angeordnet sein, so dass sich die Rohlinge 101-1, ..., 101-n nahe an der Herstellungsmaschine befinden. Mehrere Diskmanager 100 können auf mehrere Herstellungsmaschinen 113 aufgeteilt werden. Der Diskmanager 100 kann in die Herstellungsmaschine 113 beispielsweise in einer Seitenwand eingebaut sein, in der ein Arm den Rohling 101-1, ..., 101-n greift oder ein Magazin in der Vertikalen oder Horizontalen verfahrbar gelagert ist. Damit kann zu einer Übergabeposition gefahren werden, um den Rohling 101-1, ..., 101-n mit einem Roboterarm zu entnehmen.

Durch das adaptive Design des Diskmanagers 100 ist es außerdem möglich, neben den Rohlingen 101-1, ..., 101-n als solche auch Diskhalter mit eingesetzten Rohlingen zu lagern. Der Diskmanager 100 oder ein Modul 109 kann auch speziell für die Aufnahme von Diskhaltern mit eingesetzten Rohlingen 101-1, ..., 101-n ausgebildet sein. Hierbei lassen sich auch unterschiedliche Module miteinander kombinieren. Eine Information darüber, ob sich ein Rohling 101-1, ..., 101-n im Diskhalter befindet, kann in einem RFID-Tag an dem Diskhalter gespeichert sein.

Zudem ist es nicht mehr nötig, eine schriftliche Materialbezeichnung an dem Rohling zu verwenden. Dies ist hilfreich, da die schriftliche Materialbezeichnung an dem Rohling 101-1, ..., 101-n manchmal weggefräst wird. Die Lesbarkeit der Beschriftungen an den Rohlingen 101-1, ..., 101-n wird durch starke Benutzung verringert.

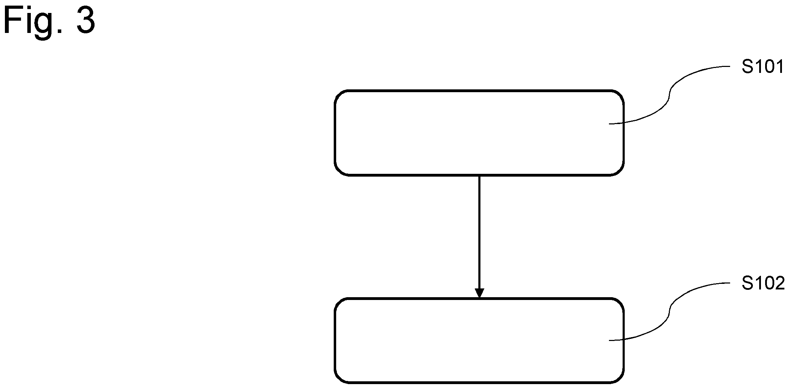

Fig. 3 zeigt ein Blockdiagramm zum Verwalten von dentalen Rohlingen 101-1, ..., 101-n. Das Verfahren umfasst den Schritt S101 eines Aufnehmens des dentalen Rohlings 101-1, ..., 101-n in eines von mehreren Aufnahmefächern 103-1, ..., 103-n. In Schritt S102 wird das Aufnahmefach 103-1, ..., 103-n angezeigt, in dem ein dentaler Rohling 101-1, ..., 101-n mit vorgegebenen Eigenschaften aufgenommen ist.

Werden mehrere Herstellungsmaschinen 113 und Diskmanager 100 verwendet, können diese auch über die Beleuchtung farbcodiert miteinander kommunizieren. Zudem kann auch eine Kommunikation mit einem Lager oder Lagerschrank durchgeführt werden. Zum Beispiel ist eine erste Herstellungsmaschine 113 mit der Beleuchtungsfarbe blau und eine zweite Herstellungsmaschine 113 mit der Beleuchtungsfarbe grün codiert. Auf diese Weise kann ein Benutzer unmittelbar erkennen, in welche Herstellungsmaschine 113 der jeweilige Rohling 101-1, ..., 101-n eingelegt werden soll. Allerdings kann der Diskmanager 100 fest einer bestimmten Herstellungsmaschine 113 zugeordnet werden, so dass diese nur Aufträge von einer bestimmten Herstellungsmaschine 113 erhält.

Die Kommunikation mit dem Lager erfolgt an einem Computer. Durch Anbindung des Diskmanagers 100 an die Herstellungsmaschine 113 können damit die lagernden Materialien überprüft werden. Der Diskmanager 100 kann ein eigenes Display aufweisen. Auch das Display der Herstellungsmaschine 113 oder das Aufrufen einer Webseite über ein mobiles Gerät kann dazu verwendet werden, um dem Benutzer Informationen anbieten zu können.

Durch den Diskmanager 100 kann auch ein ungeübter Benutzer erkennen, welcher Rohling gerade für eine Herstellungsmaschine 113 benötigt wird und diesen in die Herstellungsmaschine 113 laden, da das jeweilige Aufnahmefach 103-1, ..., 103-n in dem Modul 109 beleuchtet wird. Der Diskmanager 100 bietet also eine benutzerfreundliche Bedienung an. Neubestellungen von Rohlingen können automatisch oder regelmäßig durch den Diskmanager 100 erfolgen, da dieser immer den aktuellen Lagerbestand prüft. Beispielsweise bestellt der Diskmanager 100 bei Unterschreiten einer vorgegebenen Mindestmenge automatisch neue Rohlinge.

Auch kann eine Materialvorhersage erfolgen und eine Statistik zur Unterstützung der Bestellungen angelegt werden. Dadurch ist immer ein Mindestbestand des Lagers sichergestellt. Die Statistik kann zudem erfassen, welcher Rohling 101-1, ..., 101-n am meisten verwendet werden. Dadurch kann das Sortiment verringert werden. Ein neuer Rohling 101-1, ..., 101-n wird nur verwendet, wenn kein gebrauchter Rohling 101-1, ..., 101-n verfügbar ist. Zudem können Empfehlungen zur Lagerverwaltungsoptimierung ausgegeben werden.

Um das Nutzungsverhalten in Hinsicht auf Rohlinge oder Material des Benutzers besser zu verstehen, kann es hilfreich sein, den Diskmanager 100 in den Arbeitsablauf einzubinden, damit ein optimaler Materialfluss realisiert werden kann. Der Diskmanager 100 kann bei einem Zahnarzt oder in einem Dentallabor als Lagerautomat verwendet werden.

Alle in Verbindung mit einzelnen Ausführungsformen der Erfindung erläuterten und gezeigten Merkmale können in unterschiedlicher Kombination in dem erfindungsgemäßen Gegenstand vorgesehen sein, um gleichzeitig deren vorteilhafte Wirkungen zu realisieren.

Alle Verfahrensschritte können durch Vorrichtungen implementiert werden, die zum Ausführen des jeweiligen Verfahrensschrittes geeignet sind. Alle Funktionen, die von gegenständlichen Merkmalen ausgeführt werden, können ein Verfahrensschritt eines Verfahrens sein.

Der Schutzbereich der vorliegenden Erfindung ist durch die Ansprüche gegeben und wird durch die in der Beschreibung erläuterten oder den Figuren gezeigten Merkmale nicht beschränkt.

### BEZUGSZEICHENLISTE

- 100: Diskmanager
- 101: Rohlinge
- 103: Aufnahmefächer
- 105: Anzeigevorrichtung
- 107: Speichervorrichtung
- 109: Modul
- 111: Eingabeschnittstelle
- 113: Herstellungsmaschine
- 115: Scanvorrichtung

## Patentansprüche

1. Diskmanager (100) zum Verwalten von dentalen Rohlingen (101-1, ..., 101-n), mit:
- mehreren Aufnahmefächern (103-1, ..., 103-n) zum Aufnehmen jeweils eines dentalen Rohlings (101-1, ..., 101-n); und
- einer Anzeigevorrichtung (105-1, ..., 105-n) zum Anzeigen des Aufnahmefachs (103-1, ..., 103-n), in dem ein dentaler Rohling (101-1, ..., 101-n) aufgenommen ist.

2. Diskmanager (100) nach Anspruch 1, wobei der Diskmanager (100) eine Speichervorrichtung (107) zum Speichern der Eigenschaften des dentalen Rohlings (101-1, ..., 101-n) in dem jeweiligen Aufnahmefach (103-1, ..., 103-n) umfasst.

3. Diskmanager (100) nach Anspruch 2, wobei die Eigenschaften ein verfügbares Restmaterial, eine Farbe, eine Identifikationsnummer, eine Größe und/oder ein Herstellungsmaterial des aufgenommenen Rohlings umfassen.

4. Diskmanager (100) nach einem der vorangehenden Ansprüche, wobei der Diskmanager (100) aus mehreren Modulen (109-1, ..., 109-n) mit zumindest einem Aufnahmefach (103-1, ..., 103-n) zusammensetzbar ist.

5. Diskmanager (100) nach einem der vorangehenden Ansprüche, wobei der Diskmanager (100) eine Eingabeschnittstelle (111) zum Eingeben von Eigenschaften eines dentalen Rohlings (101-1, ..., 101-n) umfasst.

6. Diskmanager (100) nach einem der vorangehenden Ansprüche, wobei die Aufnahmefächer (103-1, ..., 103-n) eine Neigung oder eine Schaumstoffeinlage zur stabilen Positionierung des dentalen Rohlings (101-1, ..., 101-n) umfassen.

7. Diskmanager (100) nach einem der vorangehenden Ansprüche, wobei der Diskmanager (100) eine Scanvorrichtung (115) zum Scannen eines dentalen Rohlings (101-1, ..., 101-n) umfasst.

8. Diskmanager (100) nach Anspruch 7, wobei die Scanvorrichtung (115) eine optische Kamera, einen RFID-Leser, einen Bar-Code-Leser und/oder einen QR-Code-Leser umfasst.

9. Diskmanager (100) nach einem der vorangehenden Ansprüche, wobei jedem Aufnahmefach (103-1, ..., 103-n) eine Antenne (103-1, ..., 103-n) oder ein Sensor zum Erfassen des aufgenommenen dentalen Rohlings (101-1, ..., 101-n) zugeordnet ist.

10. Diskmanager (100) nach einem der vorangehenden Ansprüche, wobei jedem Aufnahmefach (103-1, ..., 103-n) eine Anzeigevorrichtung (105-1, ..., 105-n) zugeordnet ist.

11. Diskmanager (100) nach einem der vorangehenden Ansprüche, wobei die Anzeigevorrichtung (105-1, ..., 105-n) eine Leuchtdiode, eine Flüssigkristallanzeige oder ein elektronisches Papier umfasst.

12. Modul (109-1, ..., 109-n) für einen Diskmanager (100) mit zumindest einem Aufnahmefach (103-1, ..., 103-n) zum Aufnehmen eines dentalen Rohlings (101-1, ..., 101-n).

13. Modul (109-1, ..., 109-n) nach Anspruch 13, wobei das Modul (109-1, ..., 109-n) eine Anzeigevorrichtung (105-1, ..., 105-n) zum Anzeigen des Aufnahmefachs (103-1, ..., 103-n) umfasst.

14. Modul (109-1, ..., 109-n) nach Anspruch 13 oder 14, wobei das Modul (109-1, ..., 109-n) horizontal oder vertikal koppelbar ist.

15. Verfahren zum Verwalten von dentalen Rohlingen (101-1, ..., 101-n), mit den Schritten:
- Aufnehmen (S101) eines dentalen Rohlings (101-1, ..., 101-n) in einem von mehreren Aufnahmefächern (103-1, ..., 103-n); und
- Anzeigen (S102) des Aufnahmefachs (103-1, ..., 103-n), in dem ein dentaler Rohling (101-1, ..., 101-n) mit vorgegebenen Eigenschaften aufgenommen ist.

16. Verfahren nach Anspruch 15, wobei erfasst wird, ob ein dentaler Rohling (101-1, ..., 101 n) in einem der Aufnahmefächer (103-1, ..., 103-n) eingesetzt ist.

17. Verfahren nach Anspruch 15 oder 16, wobei die Restmenge eines dentalen Rohlings (101-1, ..., 101 n) erfasst wird.

18. Verfahren nach Anspruch 17, wobei ein dentaler Rohling (101-1, ..., 101 n) automatisch nachbestellt wird, wenn die Restmenge einen vorgegebenen Wert unterschreitet.

19. Verfahren nach einem der Ansprüche 15 bis 18, wobei ein Lagerbestand dentaler Rohlinge (101-1, ..., 101 n) verwaltet wird.
